## Europäisches Patentamt

## European Patent Office

## Office européen des brevets

(19)

(11) Veröffentlichungsnummer: **0 157 910**
**B1**

(12)

# EUROPÄISCHE PATENTSCHRIFT

(45) Veröffentlichungstag der Patentschrift:
**12.07.89**

(21) Anmeldenummer: **84113794.6**

(22) Anmeldetag: **10.03.82**

(51) Int. Cl.⁴: **A 61 K 9/22,** A 61 L 17/00, A 61 M 31/00

(54) Pharmakahaltige Körperchen.

(30) Priorität: **19.03.81 DE 3110805**
**14.07.81 DE 3127696**

(43) Veröffentlichungstag der Anmeldung:
**16.10.85 Patentblatt 85/42**

(45) Bekanntmachung des Hinweises auf die Patenterteilung:
**12.07.89 Patentblatt 89/28**

(84) Benannte Vertragsstaaten:
**AT CH DE FR GB LI NL SE**

(56) Entgegenhaltungen:
**DE-B-2 320 373**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Härle, Anton, Dr., Drechsler Weg 40, D-4400 Münster- Roxel (DE)**

EP 0 157 910 B1

## Beschreibung

Pharmakahaltige Körperchen, bestehend aus einem biologisch inerten Kunststoff, wobei ein oder verschiedene Pharmaka in gleichförmiger Verteilung freisetzbar in dem Kunststoff vorliegen und wobei in die Körperchen Drähte oder Fäden eingearbeitet sind.

Aus der DE-OS-2 320 373 ist ein antibiotikahaltiges Mittel in Kugelform bekannt, wobei die Kugeln mit Hilfe von Fäden oder Drähten miteinander verbunden sind. Derartige antibiotikahaltige Hilfsmittel werden in weitem Umfang in der Knochenchirurgie eingesetzt, insbesondere zur Behandlung von posttraumatischer Osteomyelitis. Die miteinander durch einen Faden oder einen dünnen Draht verbundenen Kugeln werden dabei in osteomyelitische Höhlen eingelegt.

Schwierigkeiten bestehen bei der Anwendung der bekannten Hilfsmittel dann, wenn die mit einem Draht verbundenen Körperchen wieder aus dem Körper entfernt werden sollen. Beim Herausziehen der von Bindegewebe umschlossenen Kugelkette muß eine erhebliche Kraft aufgewendet werden. Dabei kann es zum Abrutschen einzelner Kugeln vom Verbindungsdraht kommen. Die im Körper zurückgebliebenen Kugeln müssen dann in einer erneuten Operation entfernt werden.

Aus der DE-PS-2 806 609 ist es bekannt, Osteosynthesehilfsmittel mit zur Außenseite hin offenen Aufnahmeräumen für die Aufnahme und Halterung einer Trägermasse zu versehen, wobei in die Trägermasse Antibiotika eingefüllt sind. Gemäß diesem Vorschlag sind in besonderer Weise ausgebildete Osteosynthesehilfsmittel erforderlich oder bei Verwendung bekannter Osteosynthesehilfsmittel müssen die vorhandenen Ausnahmeräume vor der Operation mit dem pastösen antibiotikahaltigen Polymethacrylat oder Polyacrylat gefüllt werden, wobei bis zum Einsatz dieser Hilfsmittel dann die Zeitspanne abgewartet werden muß, in der das Polymethacrylat oder Polyacrylat aushärtet. Die Dosiskontrolle bei einer derartigen Verfahrensweise ist schwierig.

Auch diese Handhabung wurde als zu zeitaufwendig empfunden und durch die Sonderbauteile wurde der Einsatz des neuen Hilfsmittels erheblich erschwert.

Der Erfindung liegt die Aufgabe zugrunde, das an sich aus der DE-PS-2 320 373 bekannte pharmakahaltige Körperchen so in seiner Gestaltung zu verbessern, daß eine leichte, sichere und vollständige Wiederentfernbarkeit aus dem Körpergewebe, Körperhöhlen, Wunden oder dergleichen erreicht wird, die sowohl bei dem Einsatz des Körperchens unmittelbar in Wunden oder Knochenhöhlen vorhanden ist, wie auch beim Einsatz des Körperchens in Verbindung mit bekannten Osteosynthesehilfsmitteln und Implantaten.

Diese der Erfindung zugrundeliegende Aufgabe wird durch die in den Ansprüchen genannten Merkmale gelöst.

Die Körperchen sind durch eingearbeitete Fäden oder Drähte entsprechend der Erfindung miteinander verbunden, wodurch erreicht wird, daß sie auch dann, wenn bereits Bindegewebe in den Zwischenräumen gewachsen ist, problemlos entfernt werden können, da durch die eingearbeiteten Fäden oder Drähte die Körperchen nunmehr auch zugfest so fest miteinander verbunden sind, daß ein Abreißen der Körperchen in der Körperhöhle oder in der Knochenhöhle nicht mehr eintreten kann.

Es entfällt damit die sonst gegebenenfalls zusätzlich erforderliche präparatorische Entfernung, die bei den bisher bekannten Ketten auch bei sorgfältigster, chirurgischer Technik oft nicht vollständig gelingt.

Nach einer bevorzugten Ausgestaltung weisen die Körperchen wenigstens an einem Ende eine sich verjüngende Form auf. Dadurch wird ein besseres Gleiten im Körpergewebe im Vergleich zu den bisher bekannten Kugeln erreicht. Die Entfernung der Körperchen aus dem Gewebe wird dadurch erleichtert und es wird sichergestellt, daß ein vollständiges Entfernen der Körperchen möglich ist, ohne daß im Gewebe Partikelchen zurückbleiben.

Die kugelelliptische Form schafft weiterhin eine große Oberfläche für die Freigabe des Pharmakon.

Ausführungsbeispiele der Erfindung werden nachfolgend anhand der Zeichnung erläutert. Die Zeichnungen zeigen dabei in

Fig. 1 eine Ansicht miteinander druck- und zugfest verbundener Körperchen mit zusätzlich eingezogenem Draht oder Faden und mit zwei unterschiedlichen Formen in

Fig. 2 Körperchen, die durch ihre Formgestaltung die Entnahme aus dem Gewebe erleichtern und die vorzugsweise unmittelbar in Wundöffnungen, Knochenhöhlen, Wunden od. dgl. untergebracht werden.

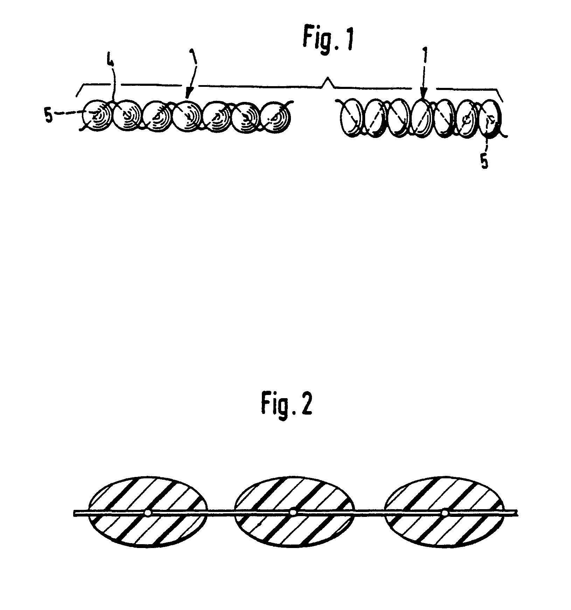

Bei dem in Fig. 1 dargestellten Ausführungsbeispiel sind die kugelförmigen oder elliptischen Körperchen 1 durch ein zusätzliches Verbindungsmittel 4 miteinander verbunden, wobei die Körperchen selbst aber wiederum materialschlüssig oder durch Verkleben miteinander verbunden sind. Hierbei kann vorzugsweise so vorgegangen werden, daß das Verbindungsmittel 4 im Endbereich der vorgesehenen Körperchenkette mit zusätzlichen Halterungsmitteln 5 ausgerüstet ist, so daß bei einem Zug an der Gesamtkette und bei einem Lösen der Körperchen voneinander die ersten und letzten Körperchen auf jeden Fall fest an dem Verbindungsmittel gehalten werden.

Die Erfindung ist selbstverständlich nicht auf die dargestellten Ausführungsbeispiele beschränkt, sondern es sind demgegenüber sowohl in der Form der Körperchen, wie auch in der Art der Verbindung der Körperchen untereinander Abänderungen möglich, ohne daß dadurch der Rahmen der Erfindung verlassen wird.

Bei dem in Fig. 2 dargestellten Ausführungsbeispiel weist das Körperchen die Form eines Rotationsellipsoides auf. Hierdurch werden spitz zulaufende Endflächen geschaffen, die die Entfernung des Körperchens durch Herausziehen od. dgl. erleichtern und ermöglichen. Die Körperchen sind dabei über Drähte oder Fäden miteinander verbunden, in die zusätzliche Halterungsmittel eingearbeitet sein können. Die Oberfläche wird gegenüber der Kugelform erheblich vergrößert.

## Patentansprüche

1. Pharmakahaltige Körperchen, bestehend aus einem biologisch inerten Kunststoff, wobei ein oder verschiedene Pharmaka in gleichförmiger Verteilung freisetzbar in dem Kunststoff vorliegen und wobei in die Körperchen Drähte oder Fäden eingearbeitet sind, dadurch gekennzeichnet, daß die Fäden oder Drähte mit zusätzlichen, ein Abrutschen der Körperchen verhindernden Halterungsmitteln versehen sind.

2. Pharmakahaltige Körperchen nach Anspruch 1, dadurch gekennzeichnet, daß die Körperchen wenigstens an einem Ende eine sich verjüngende Formgestaltung aufweisen.

3. Pharmakahaltige Körperchen nach Anspruch 1 oder 2, dadurch gekennzeichnet, daß die Körperchen rotationssymmetrisch ausgebildet sind.

4. Pharmakahaltige Körperchen nach einem der Ansprüche 1 - 3, dadurch gekennzeichnet, daß es sich bei den Pharmaka um antibakteriell wirksame Substanzen handelt.

5. Pharmakahaltige Körperchen nach Anspruch 4, dadurch gekennzeichnet, daß es sich bei den Pharmak um Substanzen aus der Gruppe der Antibiotika handelt.

6. Pharmakahaltige Körperchen nach einem der Ansprüche 1 - 3, dadurch gekennzeichnet, daß es sich bei den Pharmaka um antiviral wirksame Substanzen handelt.

7. Pharmakahaltige Körperchen nach einem der Ansprüche 1 - 3, dadurch gekennzeichnet, daß es sich bei den Pharmaka um Substanzen handelt, die gegen Gewebegeschwülste wirksam sind.

8. Pharmakahaltige Körperchen nach einem der Ansprüche 1 - 7, dadurch gekennzeichnet, daß Pharmaka mit unterschiedlichen Wirkungen in Kombinationen zur Anwendung kommen.

## Claims

1. Drug-containing particles, composed of a biologically Inert plastic, one or more drugs being releasably present in uniform distribution in the plastic, and wires or threads being incorporated in the particles, characterized in that the threads or wires are provided with additional holding means preventing the particles slipping off.

2. Drug-containing particles according to Claim 1, characterized in that the particles exhibit a tapering shaping at at least one end.

3. Drug-containing particles according to Claim 1 or 2, characterized in that the particles are designed with rotational symmetry.

4. Drug-containing particles according to one of Claims 1 to 3, characterized in that the drugs are substances with antibacterial activity.

5. Drug-containing particles according to Claim 4, characterized in that the drugs are substances from the group of antibiotics.

6. Drug-containing particles according to one of Claims 1 to 3, characterized in that the drugs are substances having antiviral activity.

7. Drug-containing particles according to one of Claims 1 to 3, characterized in that the drugs are substances which are active against tissue neoplasms.

8. Drug-containing particles according to one of Claims 1 to 7, characterized in that drugs having different effects are used in combinations.

## Revendications

1. Corpuscules renfermant des médicaments, constitués d'une matière synthétique biologique inerte où un ou différents médicaments libérables se trouvent régulièrement répartis dans la matière synthétique et où des fils ou fils métalliques sont insérés dans les corpuscules, caractérisés en ce que les fils ou fils métalliques sont munis de moyens d'attache supplémentaires empêchant un détachement des corpuscules.

2. Corpuscules renfermant des médicaments selon la revendication 1, caractérisés en ce que les corpuscules présentent au moins à une extrémité une forme allant en s'effilant.

3. Corpuscules renfermant des médicaments selon la revendication 1 ou 2, caractérisés en ce que les corpuscules ont une forme à symétrie de révolution.

4. Corpuscules renfermant des médicaments selon l'une des revendications 1 à 3, caractérisés en ce que les médicaments dont il s'agit sont constitués par des substances ayant une activité antibactérielle.

5. Corpuscules renfermant des médicaments selon la revendication 4, caractérisés en ce que les médicaments dont il s'agit sont constitués par des substances appartenant au groupe des antibiotiques.

6. Corpuscules renfermant des médicaments selon l'une des revendications 1 à 3, caractérisés en ce que les médicaments dont il s'agit sont constitués par des substances ayant une activité antivirale.

7. Corpuscules renfermant des médicaments selon l'une des revendications 1 à 3, caractérisés en ce que les médicaments dont il s'agit sont constitués par des substances actives contre les tumeurs du tissu.

8. Corpuscules renfermant des médicaments selon l'une des revendications 1 à 7, caractérisés en ce que des médicaments ayant des actions différentes sont utilisés en combinaison.

## Fig. 1

## Fig. 2